Europäisches Patentamt

European Patent Office

Office européen des brevets

(11) Publication number: **0 245 546**
**A1**

## (12) EUROPEAN PATENT APPLICATION

(21) Application number: **86303589.5**

(22) Date of filing: **12.05.86**

(51) Int. Cl.⁴: **A01H 1/02** , B07B 4/08

(43) Date of publication of application:
**19.11.87 Bulletin 87/47**

(84) Designated Contracting States:
**AT BE CH DE FR GB IT LI LU NL SE**

(71) Applicant: **THE DIRECTOR GENERAL OF THE MINISTRY OF AGRICULTURE AND FISHERIES Gillingham House 101-103 The Terrace Wellington(NZ)**

(72) Inventor: **Stevenson, Barry Ernest 130 Silverdale Road Hamilton(NZ)**

(74) Representative: **Boyes, Kenneth Aubrey et al Frank B. Dehn & Co. Imperial House 15-19 Kingsway London WC2B 6UZ(GB)**

(54) **Drying and separating material.**

(57) The apparatus comprises a chamber (11) for holding pollen bearing anthers, which has an inlet (5) for air and an outlet (15) from the chamber (11). The outlet (15) is spaced apart from the inlet (5) and allows for air to be drawn or forced through the inlet (5) and to pass through the chamber (11) to exit from the outlet (15). The apparatus is characterised in that air issuing from the inlet (5) passes through means (18) having a plurality of openings or bores (20) therein, said openings (20) each being disposed at an angle relative to the direction of incoming air, such that air issuing through the openings or bores (20) and into the chamber (11) has a substantially twisting motion. The method comprises the steps of placing pollen bearing anthers into a chamber (11), and passing a twisting stream of air through said chamber (11), such as to disturb the anthers and release pollen therefrom.

FIG 1

## "Drying and Separating Material"

This invention relates to a method and apparatus for drying and separating material.

To enable artificial pollination of fruit producing plants, trees, vines, bushes, and the like to be carried out, it is necessary to firstly extract pollen from the flowers. This usually involves milling the flowers in order to remove the anthers therefrom and then drying the anthers and "disturbing" them in order to release the pollen stored therein. With research into artificial pollination of certain fruit bearing plants such as, for example, kiwifruit there is a need to be able to recover pollen in such a manner that the pollen has a high degree of viability.

Whilst it is possible to achieve recovery of pollen of high viability using stationary anthers, driers and subsequent pollen separation, such a method is not commercially acceptable due to the time taken to collect relatively small yields of pollen. Other known apparatus are not suitable for drying anthers for one reason or another, however, in the main, known apparatus is unsuitable because of the damage caused during the drying and/or "disturbing" process thereby resulting in pollen of low viability.

A known apparatus for the drying of materials is one which operates on a fluid bed principle whereby the material to be dried is placed within a chamber through which air is passed. Usually, the chamber is vertically orientated and the air is forced through an opening, or more usually, openings in the base of the chamber. This air flow through the chamber is ducted from the upper end thereof and passed to a separator, such as a cyclone, whereby the dried material entrained in the air is removed.

A conventional fluid bed drier is, however, unsuitable for drying anthers and separating pollen therefrom, especially anthers taken from the male flowers of a kiwifruit vine, as the anthers have long filaments that tend to entangle with each other thereby causing the anthers to "nest" in lumps. Accordingly, in such a conventional fluid bed the air tends to blow one or two holes through the bed of anthers thereby resulting in unsatisfactory drying of the pollen.

It is an object of the present invention to provide an apparatus and method suitable for both drying anthers and separating pollen therefrom, which permits effective drying and separating, but which avoids, or at least substantially minimises, anthers becoming entangled and "nesting" into lumps.

According to one aspect of this invention there is provided apparatus for drying anthers and separating pollen contained therein, said apparatus comprising a chamber for holding pollen bearing anthers; an inlet for admission of air; an outlet from the chamber; said outlet being spaced apart from said inlet; whereby air forced or drawn through said inlet is able to pass through the chamber to exit therefrom through said outlet; characterised in that air issuing from said inlet passes through means having a plurality of openings or bores therein, said openings or bores being disposed at an angle such that air issuing through said openings and into said chamber has a substantially twisting motion.

According to a further aspect of this invention there is provided a method of drying anthers and separating pollen contained therein, which comprises the steps of placing pollen bearing anthers into a chamber, and thereafter passing a twisting tream of air through said chamber so as to disturb said anthers and to release pollen therefrom.

According to a further aspect of this invention, there is provided an apparatus for drying anthers and separating pollen contained therein, comprising a chamber for holding pollen bearing anthers; an inlet for admission of air and an outlet from said chamber; characterised in that means are provided inwardly of said air inlet to impart a substantially twisting motion to air issusing into said chamber.

An embodiment of the present invention will now be described by way of example only, and with reference to the accompanying drawings, wherein:

Figure 1 is an elevational view of one embodiment of apparatus according to the present invention,

Figure 2 is a plan view of a base plate of the apparatus of the embodiment shown in Fig. 1, and

Figure 3 is an elevational view showing air flow through the base plate shown in Figure 2.

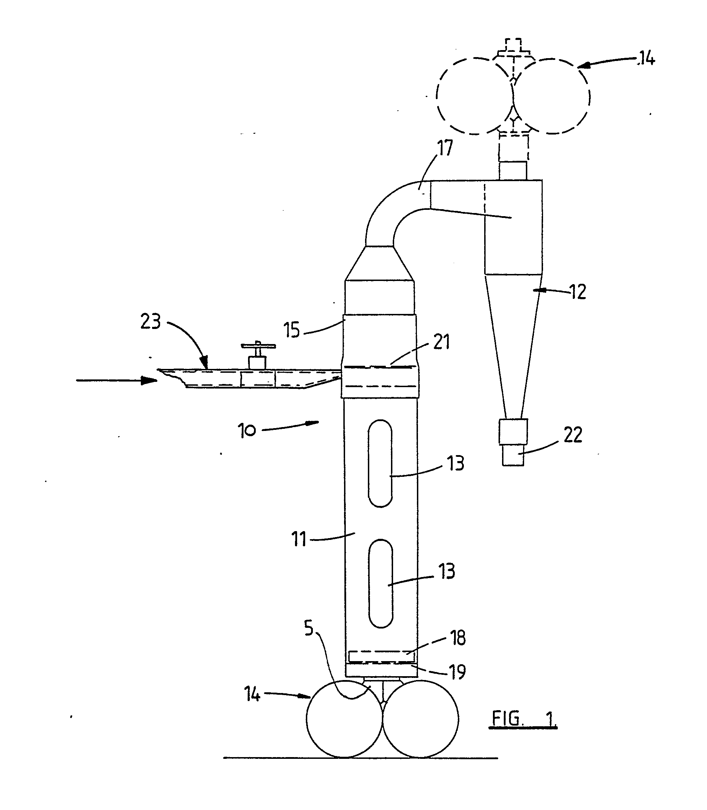

Referring firstly to Figure 1 of the accompanying drawings, there is illustrated a drying apparatus 10, which includes a tower 11 connected by appropriate connecting means, to a cyclone 12. In a preferred embodiment, the tower 11 is conveniently provided with one or more viewing windows or ports 13.

The lower end of the tower 11 is connected to an appropriate air supply, for example a multi-stage fan air supply 14, whereby air can be forced into an inlet 5 at the base of the tower 11. This is however by way of example only, and any appropriate air supply means can be provided.

In use, the air passes through the tower 11 to an outlet arrangement 15 and is then ducted, by way of ducting 17, to the cyclone 12.

In Figure 1, an alternative arrangement is shown in ghosted detail, whereby an air supply 14 is located at or adjacent the air exit 12, where it draws or sucks air through an inlet 5 and through the tower 11.

Within the tower 11, spaced apart from, and preferably located above the inlet 5, an appropriate base plate 18 is provided. The base plate 18 is preferably fixedly mounted within the tower 11. It can however be movably (i.e.: rotatably) mounted if desired.

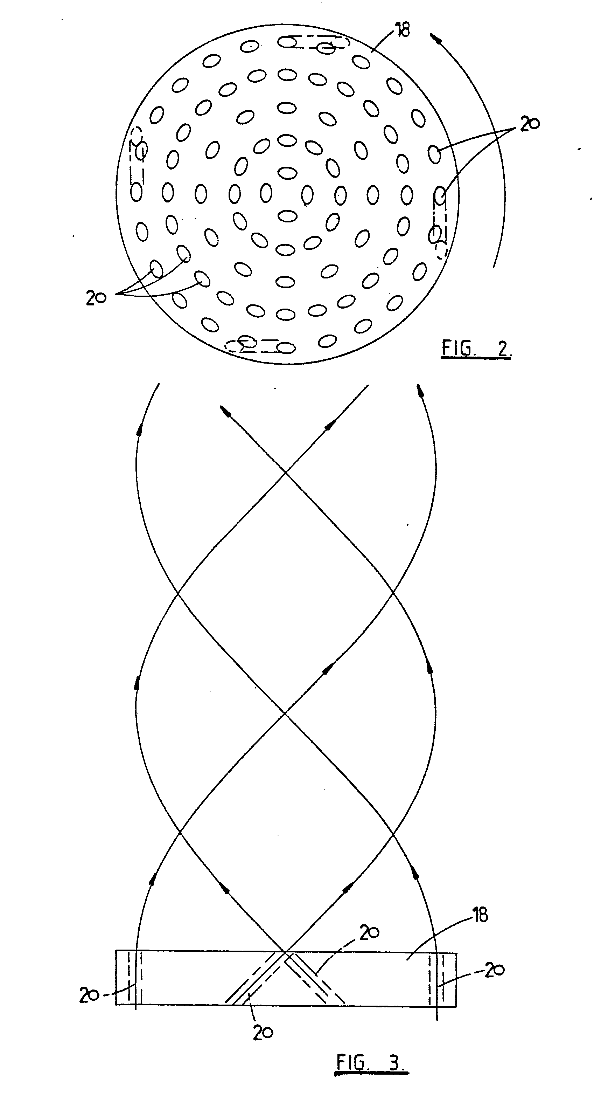

Referring in particular to Figures 2 and 3 the base plate 18, which can be constructed of any appropriate material, has a plurality of air entry openings or ports 20, which preferably extend therethrough at an angle to the horizontal.

In one embodiment, as shown for example in Figure 1 of the accompanying drawings, a mesh filter element 19 can be provided adjacent and preferably below the base plate 18.

The base plate 18 is preferably suitably located and housed within the tower 11 and, in a preferred embodiment, the base plate 18 is fored and designed so as to be suitable for the drying and separating of pollen from kiwifruit anthers.

In the preferred embodiment the openings or holes 20 are formed so as to be at a typical angle of approximately 45° to the horizontal.

While is the preferred embodiment the openings or bores 20 are substantially circular or oval when viewed in plan, and at an angle of approxiamtely 45° to the horizontal, it should be appreciated that other angles are possible, to obtain satisfactory results from the apparatus and method.

It will be appreciated by those skilled in the art, that the size, spacing and angles of the openings 20, will be open to change and variations, in order to gain different drying and separating effects.

In the preferred embodiment however, and as illustrated in Figures 2 and 3 of the accompanying drawings, the openings 20 are located in concentric circles, substantially concentric with the centre of the base plate 18. The openings all slope or angle, for example, in the direction of the arrow shown in Figure 2 of the drawings. It should be appreciated that this arrow also indicates the direction of twisting of the air stream which passes through the plate 18. This will be described further hereinafter.

While in the preferred embodiment the openings or bores 20 are formed so as to slope or angle in substantially the same direction, (such as for example shown in Figure 2 of the accompanying drawings), the openings or bores 20 may, for example, slope or be angled in different directions.

The plate 18 is preferably of such a thickness that the openings or bores 20 are elongate in formation.

The apparatus also includes a screen 21, which is preferably situated above the chamber of the tower 11 and at or adjacent the outlet 15. In the preferred embodiment, the screen 21 is provided within the outlet arrangement 15 and is in the form of a 100 micron screen. Such a screen, while being referred to by way of example only, has been found to be particularly advantageous in separating pollen from kiwifruit anthers.

An air nozzle 23 can in the preferred embodiment be attached or fitted in an appropriate known manner, to clean the screen. In the preferred embodiment, means are provided whereby the nozzle 23 will provide an intermittent air stream relative to the screen 21, so that the screen 21 is cleaned intermittently.

Appropriate collection means, such as for example a pollen collection container 22, is located on or attached to the cyclone 12.

In use, kiwifruit anthers from from the male kiwifruit flower, either in a "raw" condition or prior dried (for example in a stationary dryer), are placed into the chamber of the tower 11. Air from the air supply 14 is formed or drawn into the chamber through an opening at the base of the chamber of the tower 11. This air flow passes through the base plate 18, by way of openings or bores 20, and due to the slanting or angled sides of the openings or bores 20 relative to the direction of incoming air, the air flow is given, or has imparted thereto, a positive "twist". This twisting air flow which is - schematically illustrated by way of example only, in Figure 3 of the drawings, grips and pulls the "nesting" anthers apart in what is an effective and yet relatively gentle manner. Thus, the method does not result in any significant pollen damage.

Pollen released from the anthers is extracted from the air stream by appropriate means; for example by using the cyclone 12. The screen 21 assists in preventing any anthers from entering into the cyclone. The pollen then exits into the container 22, for collection, storage and use.

Should a "blow-hole" in the nest of anthers begin to develop, the twisting air being applied to the anthers, will break up the anthers surrounding the hole allowing even drying to take place and continue. Because the air flow mixes the anther load continually, substantially even drying occurs throughout the full height of the load. It has been found therefore that the load does not dry in layers, which would form and result in bottom layers being overdried while top layers remained undried, or substantially undried.

In trials conducted with kiwifruit anthers, pollen having a viability ranging from between approximately 82.1% and 87.5%, (depending upon the rate of air entry into the chamber), was extracted, using the method and apparatus of the preferred embodiment.

In one embodiment, the air supply 14 is provided with and/or connected to, an appropriate heating arrangement, whereby air is heated prior to being forced or drawn through the inlet and into the chamber. Suitable control means or thermostat means can be associated therewith, so as to control the temperature of such air.

In a further embodiment, it is envisaged that the base plate 18 is in the form of a plate having a series of nozzles attached or affixed thereto. Suitable holes or bores are provided in the plate to admit air into the nozzles. The nozzles are suitably angled or directed so as to provide or impart the desired twisting motion to the air.

It is envisaged that other means may be provided to impart the substantially twisting motion to the air passing through the chamber.

The apparatus and method described hereinbefore are, in one embodiment, particularly suitable for separating pollen from anthers which are prior dried in a separate drying arrangement, such as for example a stationary drier. This is however by way of example only.

Thus it will be seen that, at least in its preferred forms the invention provides an apparatus and method for drying anthers and separating pollen therefrom, which goes at least some way towards overcoming or minimising the problems outlined above providing an efficient method of drying anthers and separating pollen therefrom and which provides the public and industry with a useful choice.

Throughout the above description, reference has been made by way of example, to the drying and separating of pollen from anthers, and to a method of recovering pollen from pollen bearing anthers. In particular to the recovery of pollen from anthers taken from the male flower of kiwifruit vines. It should be appreciated however, that this is by way of example only, and that the apparatus and method of the present invention are equally applicable to anthers taken from other plants, bushes, trees, vines, vegetation and the like. Indeed, the present invention is applicable to any particulate matter which would normally be subjected to satisfactory drying in a standard fluid bed dryer, in which the particulate material normally binds or otherwise joins together, while in the air flow of the dryer.

## Claims

1. Apparatus for drying anthers and separating pollen contained therein, said apparatus comprising a chamber (11) for holding pollen bearing anthers, an inlet (5) for admission of air, an outlet (15) from the chamber (11); said outlet (15) spaced apart from said inlet (5), whereby air forced or drawn through said inlet (5) passes through the chamber (11) to exit therefrom through said outlet (15); characterised in that air issuing from said inlet (5) passes through means (18) having a plurality of openings or bores (20) therein, said openings (20) being slanted or disposed at an angle such that air issuing through said openings (20) and into said chamber (11) has a substantially twisting motion.

2. Apparatus as claimed in claim 1, comprising extraction means (12) to extract pollen from an air stream exiting from said outlet (15).

3. Apparatus as claimed in claim 1 or 2, wherein said openings (20) are arranged in a series of concentric circles.

4. Apparatus as claimed in claim 1, 2 or 3 wherein said openings (20) are angled at substantially the same angle and in substantialy the same direction.

5. Apparatus as claimed in any preceding claim, wherein said means (18) having a plurality of openings (20) comprises a plate (18) disposed above said inlet (5), said openings (20) extending through said plate (18).

6. Apparatus as claimed in claim 5, wherein said plate is of a thickness such that said openings are elongate.

7. Apparatus as claimed in claim 5 or 6, wherein said openings (20) are substantially circular in cross-section and are at an angle of substantially 45° to the horizontal axis of said plate (18).

8. Apparatus as claimed in claim 5, 6 or 7, wherein a mesh screen (19) is disposed below said plate (18).

9. Apparatus as claimed in any preceding claim, wherein a mesh screen (21) is disposed adjacent the outlet (15) from said chamber (11).

10. Apparatus as claimed in claim 9, wherein air cleaning means (23) is associated with said screen (21).

11. Apparatus for drying anthers and separating pollen contained therein, comprising a chamber (11) for holding pollen bearing anthers; an inlet (5) for admission of air and an outlet (15) from said chamber (11); characterised in that means (18) are provided inwardly of said air inlet (5) to impart a substantially twisting motion to air issuing into said chamber.

12. A method of drying anthers and separating pollen contained therein, which comprises the steps of placing pollen bearing anthers into a chamber (11); and thereafter passing a twisting stream of air through said chamber (11) so as to disturb said anthers and release pollen therefrom.

13. A method as claimed in claim 12, comprising the further step of extracting said pollen from said air stream.

14. A method as claimed in claim 12 or 13, comprising passing said air through a plurality of spaced apart openings or bores (20) in a plate (18) located inwardly of an air inlet (5) leading into said chamber (11).

FIG. 1

FIG. 2.

FIG. 3.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | FR-A-2 328 515 (BROWN & WILLIAMSON TOBACCO CORP.)<br><br>* Figures 1,3; page 1, line 29 - page 5, line 7 * | 1,2,5, 6,8,13 ,14 | A 01 H 1/02<br>B 07 B 4/08 |
| A | BE-A- 847 326 (KELLER-PEUKERT)<br><br>* Figures 1,3,4; page 8, line 19 - page 9, line 29; page 10, line 23 - page 11, line 17 * | 1,3-5, 7,11, 12,14 | |
| A | FR-A-1 398 143 (SOCIETE BIRTLEY)<br>* Figures 1-4; page 2, left-hand column, line 30 - right-hand column, line 20 * | 4-6,14 | |
| A | US-A-4 087 937 (MEADOR) | | A 01 H<br>B 07 B<br>B 03 B |

| | TECHNICAL FIELDS SEARCHED (Int. Cl.4) |
|---|---|

The present search report has been drawn up for all claims

| Place of search | Date of completion of the search | Examiner |
|---|---|---|
| THE HAGUE | 22-12-1986 | DISSEN H.D. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1503 03 82